# EUROPEAN PATENT SPECIFICATION

(11) **EP 2 187 926 B1**
(45) Date of publication and mention of the grant of the patent: **25.01.2012**
(21) Application number: 08803975.5
(22) Date of filing: 10.09.2008
(51) Int. Cl.: A61K 38/16, A61P 3/10

(54) **Prevention of diabetes by administration of gliadin**
Vorbeugung von Diabetes durch Verabreichung von Gliadin
Prévention du diabète par l'administation de gliadine

(30) Priority: 11.09.2007 US 993327 P; 24.06.2008 US 75055
(43) Date of publication of application: 26.05.2010
(73) Proprietor: Københavns Universitet, 2200 Kobenhavn N (DK)
(72) Inventor: BUSCHARD, Karsten, DK-2920 Charlottenlund (DK); FUNDA, David, 130 00 Prague 3 (CZ); FUNDOVA, Petra, 130 00 Prague 3 (CZ); HANSEN, Axel, Kornerup, DK-4000 Roskilde (DK)
(74) Representative: Orsnes, Henrik Egede
(86) International application number: PCT/EP2008/062008
(87) International publication number: WO 2009/034110

(56) References cited:
- WO-A-2006/112925
- MAURANO ET AL: "Intranasal administration of one alpha gliadin can downregulate the immune response to whole gliadin in mouse" SCANDINAVIAN JOURNAL OF IMMUNOLOGY, vol. 53, 2001, pages 290-295, XP002502331 cited in the application
- FUNDA ET AL: "Gluten-free but also gluten-enriched (gluten+) diet prevent diabetes in NOD mice; the gluten enigma in type 1 diabetes" DIABETES/METABOLISM RESEARCH AND REVIEWS, vol. 24, 2 July 2007 (2007-07-02), pages 59-63, XP002502330 cited in the application
- SCHMID: "Delayed exposure to wheat and barley proteins reduces diabetes incidence in non-obese diabetic mice" CLINICAL IMMUNOLOGY, vol. 111, 2004, pages 108-118, XP002502334
- BARBEAU ET AL: "Putting the pieces of the puzzle together - a series of hypotheses on the etiology and pathogenesis of type 1 diabetes" MEDICAL HYPOTHESES, vol. 68, 11 October 2006 (2006-10-11), pages 607-619, XP005820990
- TOLLEFSEN ET AL: "HLA-DQ2 and -DQ8 signatures of gluten T cell epitopes in celiac disease" THE JOURNAL OF CLINICAL INVESTIGATION, vol. 116, 2006, pages 2226-2236, XP002502333
- MARIETTA ET AL: "Intranasal gliadin peptide therapy and oral feeding with gluten inhibits blistering in a mouse model of dermatitis herpetiformis", CLINICAL IMMUNOLOGY, vol. 119, 2006, page S46, XP024911297,

## Description

### FIELD OF THE INVENTION

The invention is directed to the prevention of diabetes, in particular type 1 diabetes. More specifically the invention relates to immunization against diabetes, in particular type 1 diabetes, by intranasal administration of gliadin or fragments thereof.

### BACKROUND OF THE INVENTION

Type 1 diabetes is a common disease, affecting almost 20 million people worldwide. It comes with the burden of daily insulin injection and blood testing, as well as both short- and long-term complications, and this can include premature death. Even with tight glucose control, there is a significant risk of neuropathy, retinopathy and nephropathy, as well as a 3-fold increase in the risk of severe hypoglycaemia. Type 1 diabetes accounts for approximately 15% of the diabetic population. Insulin deficiency is a result of the autoimmune destruction of the insulin producing pancreatic beta cells; however, clinical onset of diabetes does not occur until 80-90% of these cells have been destroyed.

Type 2 diabetes, formerly known as non insulin-dependent diabetes mellitus (NIDDM), accounts for the majority of the remainder of the diagnosed cases of diabetes. Type 2 diabetes stems from both a decreased secretion of insulin as well as the body's inability to effectively utilize the insulin produced (insulin resistance). Current research suggests that the disposition to Type 2 diabetes is genetically inherited, with a high concordance rate in identical twins. Also referred to as adult-onset diabetes, Type 2 diabetes generally develops after the age of thirty, and is commonly associated with obesity. However, Type 2 diabetes can develop at an earlier age. Important is that several immunological components have been identified in the pathogenesis of the disease in many of the sub-groups of diabetes, including LADA, Moody and gestational diabetes.

Histological analysis of the pancreas from patients with type 1 diabetes shows immunological activity not present in a healthy or a type 2 diabetic pancreas (Foulis AK, Farquharson MA, Hardman R. Aberrant expression of Class II major histocompatibility complex molecules by B cells and hyperexpression of Class I major histocompatibility complex molecules by insulin containing islets in Type 1 (insulin-dependent) diabetes mellitus. Diabetologia 1987; 30:333-343). This activity is limited to insulin-containing islets, includes infiltration by activated lymphocytes, antibodies, (including glutamic acid decarboxylase (GAD), IA-2, and insulin (IAA)) and components of the complement system. This inflammation is called insulitis. These histological findings are consistent with type 1 diabetes being an immune-mediated disease.

Further evidence of type 1 diabetes being an immune-mediated disease comes from studies showing that drugs that suppress the immune response can modulate type 1 diabetes. Drugs such as cyclosporin and azathioprine slow progression of β cell destruction, and have been used in trials of type 1 diabetes prevention (Dupre J, Kolb H. Cyclosporin-induced remission of IDDM after early intervention -association of 1 yr of cyclosporin treatment with enhanced insulin secretion. Diabetes 1988; 37:1574-1582. Feutren G, Assan R, Karsenty G, Durostu H, Sirmai J, Papoz L, Vialettes B, Vexiau P, Rodier M, Lallemand A. Cyclosporin increases the rate and length of remissions in insulin- dependent diabetes of recent onset - results of a multicenter double-blind trial. Lancet 1986; 2:119-124).

Immune responses are mediated by many mechanisms including innate immunity and adaptive immune responses (antibodies, T cells). Under certain circumstances, the immune system does not produce an immune response against antigens due to a mechanism called "tolerance". Tolerance is probably an active process. Regulatory T cells and soluble factors, such as interleukin-10 (IL-10) or TGF, have been proposed in mechanisms of peripheral tolerance. The regulatory T cells are controlled by the environment, tissue cells and antigen presenting cells.

When tolerance breaks down, the organism may produce a cellular immune response (including cytotoxic T cells) to normal constituents of the organism, producing an "autoimmune disease". Examples of autoimmune diseases include rheumatoid arthritis (RA), multiple sclerosis (MS) and systemic lupus erythematosus (SLE).

Presentation of soluble antigen on mucosal surfaces, so far tested mainly via the oral route, leads to selective T-cell suppression also known as "oral tolerance", which is associated with a shift towards Th2 responses, or in case of a higher antigen doses, with T cell anergy and T cell deletion. (Faria AM, Weiner HL. Oral tolerance: mechanisms and therapeutic applications. Adv Immunol. 1999; 73:153-264. Krause I, Blank M, Shoenfeld Y. Immunomodulation of experimental autoimmune diseases via oral tolerance. Crit Rev Immunol. 2000; 20:1-16. Weiner HL. Oral tolerance: immune mechanisms and treatment of autoimmune diseases. Immunol Today. 1997; 18:335-43). Hanninen et al. (Hanninen A, Braakhuis A, Heath WR, Harrison LC. Mucosal antigen primes diabetogenic cytotoxic T-lymphocytes regardless of dose or delivery route. Diabetes. 2001 ; 50:771-5) showed that tollerogenic antigen administration also led to induction of cytotoxic T lymphocytes.

There is increasing evidence that the anti-inflammatory cytokine IL-10 plays a critical role in the development of mucosal regulatory T cells (Kaya Z, Dohmen KM, Wang Y, Schlichting J, Afanasyeva M, Leuschner F, Rose NR. Cutting edge: a critical role for IL-10 in induction of nasal tolerance in experimental autoimmune myocarditis. J Immunol. 2002; 168:1552-6. Battaglia M, Stabilini A, Draghici E, Migliavacca B, Gregori S, Bonifacio E, Roncarolo MG. Induction of tolerance in type 1 diabetes via both CD4+CD25+ T regulatory cells and T regulatory type 1 cells. Diabetes 2006; 55:1571-80). Thus, many factors such as antigen dose, administration route, T-cell precursor frequency or inflammatory co-stimulation can influence the balance between tolerance and immunity.

Intranasal administration of pharmaceutical compounds is a known method of drug delivery. Dry powder formulations, aerosol or nasal drops comprising therapeutic agents have been applied directly to the nasal membranes to achieve a rapid, local or systemic therapeutic effect.

Systemic delivery via the nasal mucosa has a number of beneficial properties. For example, the absorption of the pharmaceutically active agent into the bloodstream via the nasal mucosa can be rapid, and faster than, for example, absorption via the gastrointestinal tract following oral administration of an active agent. Nasal administration also allows "first pass" metabolism to be avoided, a problem associated with the oral administration of some pharmaceutically active agents. Further to this, it is also possible to use nasal administration for providing slower, sustained release of drugs. Intranasal administration of autoantigens has several advantages over the oral route such as: lower dose, convenient application in humans, less variability due to degradation and/or absorption. Furthermore, intranasal administration may poses a quality by itself trough the relative easy access to a immunologically important mucosal surface.

In studies dealing with type 1 diabetes models, intranasal administration of insulin and GAD65 peptides (Glutamic acid decarboxylase 65, an important autoantigen in diabetes type 1) were shown to prevent diabetes in NOD mice (Daniel D, Wegmann DR. Protection of nonobese diabetic mice from diabetes by intranasal or subcutaneous administration of insulin peptide B-(9-23). Proc Natl Acad Sci U S A. 1996; 93:956-60. Tian J, Atkinson MA, Clare-Salzler M, Herschenfeld A, Forsthuber T, Lehmann PV, Kaufman DL. Nasal administration of glutamate decarboxylase (GAD65) peptides induces Th2 responses and prevents murine insulin-dependent diabetes. J Exp Med 1996; 183:1561-7). Proinsulin peptide p24-33, also reduced diabetes incidence in NOD mice (Chen W, Bergerot I, Elliott JF, Harrison LC, Abiru N, Eisenbarth GS, Delovitch TL. Evidence that a peptide spanning the B-C junction of proinsulin is an early autoantigen epitope in the pathogenesis of type 1 diabetes. J Immunol. 2001; 167:4926-35). Whilst mucosal administration of oral insulin (Bergerot I, Fabien N, Maguer V, Thivolet C. Oral administration of human insulin to NOD mice generates CD4+ T cells that suppress adoptive transfer of diabetes. J Autoimmun. 1994; 7:655-63) or insulin peptide (Daniel D, Wegmann DR. Protection of nonobese diabetic mice from diabetes by intranasal or subcutaneous administration of insulin peptide B-(9-23). Proc Natl Acad Sci U S A. 1996; 93:956-60) leads to induction of CD4+ T regulatory cells, aerosol application of the whole insulin molecule led to induction of CD8+ gamma/delta T cells in the spleen (Harrison LC, Dempsey-Collier M, Kramer DR, Takahashi K. Aerosol insulin induces regulatory CD8 gamma delta T cells that prevent murine insulin-dependent diabetes. J Exp Med. 1996; 184:2167-74).

Two trials of intranasal insulin administration in humans at risk of type 1 diabetes have been started in Australia, intranasal insulin trial (INIT) and Finland, Diabetes Prediction and Prevention Project (DIPP), Clinical Trials sponsored by Juvenile Diabetes Research Foundation International (JDRF). While some human trials are still in progress other such as the Diabetes Prevention Trial-1 (DTP-1) with intra-venous and oral insulin failed to show a protective effect. These data together with negative outcome from human trials with oral autoantigens in other autoimmune diseases have caused a great deal of disappointment. With exception of DTP-1 study, these trials have been, however, carried out on individuals with end-stage of the disease (reviewed in Hänninen A, Harrison LC. Mucosal tolerance to prevent type 1 diabetes: can the outcome be improved in humans; Rev Diabet Stud. 2004; 1:113-121). Also the optimal dose and timing may play a critical role in human trials.

There are no approved vaccines for Type 1 diabetes on the market, but drug development companies for example Diamyd Therapeutic AB with its GAD-based product Diamyd® and DeveloGen (through a merge with Peptor) with DiaPep277® are two main competitors. This peptide is disclosed in patent application US20050152914 A1. Results show that therapeutic vaccination with p277 (a fragment of heat shock protein 60) can arrest the spontaneous diabetogenic process both in NOD mice and in humans associated with a T(h)1 to T(h)2 cytokine shift specific for the autoimmune T cells. DeveloGen and Peptor showed in trials that DiaPep277® delayed or arrested the progression of type 1 diabetes and in newly diagnosed type 1 patients. They also showed that DiaPep277® reduced the need for injected insulin (Raz I, Elias D, Avron A, Tamir M, Metzger M, Cohen IR. Beta-cell function in new-onset type 1 diabetes and immunomodulation with a heat-shock protein peptide (DiaPep277): a randomised, double-blind, phase II trial. Lancet. 2001; 358:1749-53; Raz I, Avron A, Tamir M, Metzger M, Symer L, Eldor R, Cohen IR, Elias D. Treatment of new-onset type 1 diabetes with peptide DiaPep277 is safe and associated with preserved beta-cell function: extension of a randomized, double-blind, phase II trial. Diabetes Metab Res Rev. 2007; 23:292-8). Uses for p277 as a vaccine are disclosed in patent applications WO2006072946 A2, WO 02/16549 and WO2005072056 A2.

Diamyd Medical AB is focused on developing treatments for diabetes, both Type 1 and Type 2, via GAD (Glutamic Acid Decarboxylase) protein therapy. Its lead drug candidate, Diamyd®, is designed to reduce the need of insulin injections and prevent the destruction of beta cells. The Company is conducting two clinical trials concurrently (2007) in Sweden. However, possible use of both DiaPep277® and GAD are only delaying the disease progression. In contrast to the invention herein were the vaccination is preventive i.e. given before the onset or very early of the disease.

Patients and relatives with type 1 diabetes are at increased risk of other immune mediated diseases. These most commonly include coeliac disease, thyroid disease, autoimmune gastritis and Addison's disease.

Coeliac disease is a common chronic inflammatory enteropathy, caused by dietary gluten or more specifically by gliadin. Gliadin is a glycoprotein within gluten and is found in wheat and some other grains, including rye, barley, and millet. Although the pathophysiology of coeliac disease is not completely understood, it is clear that the presence of the toxic proteins in the patient's diet causes a total or partial damage of intestinal mucosa leading to severe malabsorption syndromes and causing diarrhea, vomit, abdominal pain, anorexia, growth retard, undernutrition and anemia (Brandtzaeg, P. Mechanisms of gastrointestinal reactions to food. Environmental Toxicology and Pharmacology. 1997; 4:9-24). Coeliac disease affects children, but it is also common in adults. Coeliac disease is often undiagnosed or misdiagnosed and sometimes is clinically atypical or asymptomatic. Current treatment is by strict lifelong gluten exclusion - a difficult, socially restrictive and expensive therapy.

The association between coeliac disease and type 1 diabetes has been studied for almost 50 years. Many studies all over the world have demonstrated increased prevalence of coeliac disease in children, adolescents and adults with type 1 diabetes. Both are autoimmune conditions resulting from a complex interaction between genetic, immunological and environmental factors. It is not yet clear whether the simultaneous occurrence of the two diseases is linked to a common genetic base, or whether one disease in fact predisposes patients to the other; currently it is the first hypothesis that is more widely accepted.

In animal models, removal of dietary gluten protects against the development of type 1 diabetes (Funda DP, Kaas A, Bock T, Tlaskalová-Hogenová H, Buschard K. Gluten-free diet prevents diabetes in NOD mice. Diabetes Metab Res Rev. 1999; 15:323-327). In humans, Type 1 diabetes and other autoimmune diseases occur at a lower rate in patients diagnosed with coeliac disease at a younger age, suggesting that early elimination of gluten may protect against the manifestation of type 1 diabetes in humans (reviewed in Cronin CC, Shanahan F. Insulin-dependent diabetes mellitus and coeliac disease. Lancet 1997; 349:1096-1097). Several studies are confirming this theory, for example Fuchtenbusch et al. (Fuchtenbusch M, Ziegler AG, Hummel M. Elimination of dietary gluten and development of type 1 diabetes in high risk subjects. Rev Diabet Stud. 2004; 1:39-41) documented that exposure to dietary gluten in offspring of mothers and fathers with type 1 diabetes very early in life is associated with an increased risk of developing islet antibodies; suggesting that removal of dietary gluten should be tested as early as possible in children with an increased risk of islet autoimmunity, i.e. before an immune response to islet antigens is established.

The disclosure of the invention herein differs from the above mentioned suggestions. In the present invention an early introduction of gliadin decreases the prevalence of type 1 diabetes.

The inventors of the present invention showed that gluten-free but also gluten-enriched diet prevent diabetes in NOD mice (Funda DP, Kaas A, Tlaskalova-Hogenova H, Buschard K. Gluten-free but also gluten-enriched (gluten+) diet prevent diabetes in NOD mice; the gluten enigma in type 1 diabetes. Diabetes Metab Res Rev. 2008; 24: 59-63). Possible mechanisms of the dual effect of dietary gluten on the development of diabetes type 1 are discussed. One mechanism proposed is that gluten may influence diabetes incidence by a direct effect on the gut mucosa. Gliadin displays lectin-like properties and similarly to LPS directly stimulates innate immune responses through polyclonal activation and/or activation of NF-kappaB (Jelinkova L, Tuckova L, Cinova J, Flegelova Z, Tlaskalova-Hogenova H. Gliadin stimulates human monocytes to production of IL-8 and TNF-alpha through a mechanism involving NF-kappaB. FEBS Lett. 2004; 571:81-5. Nikulina M, Habich C, Flohe SB, Scott FW, Kolb H. Wheat gluten causes dendritic cell maturation and chemokine secretion. J Immunol. 2004; 173:1925-33). High doses or long exposure to LPS induces unresponsiveness or tolerance (Labeta MO, Durieux JJ, Spagnoli G, Fernandez N, Wijdenes J, Herrmann R. CD14 and tolerance to lipopolysaccharide: biochemical and functional analysis. Immunology 1993; 80:415-23). However they do no mention using gliadin or parts thereof for an immunization/vaccination strategy to prevent or cure type 1 diabetes.

In coeliac disease, T-cell epitopes of the gluten protein have been identified that activate T cells in the disease. Such epitopes could be used for developing a T cell vaccine or to genetically modify dietary gluten so that it represents no harm for coeliac patients (The Walter and Eliza Hall Institute of Medical Research Annual Report 2004 - 2005). However, unlike the invention herein they do not immunize with the antigen (gliadin) itself.

Our invention that mucosal administration of gliadin leads to significant prevention of type 1 diabetes have important implication for human type 1 diabetes, and also for celiac disease. Since environmental factors represent the major cause of the recent epidemiological increase of type 1 diabetes, environmental antigens related to etiology of increased penetration of type 1 diabetes in developed countries should be tested for the disease prevention. Oral administrations of gliadin leads to establishment of oral tolerance (Troncone R, Ferguson A. Gliadin presented via the gut induces oral tolerance in mice. Clin Exp Immunol. 1988; 72:284-7), however exposure to other non-danger environmental stimuli such as presence of gut microflora is required for proper development of mechanisms of oral tolerance (Wannemuehler MJ, Kiyono H, Babb JL, Michalek SM, McGhee JR. Lipopolysaccharide (LPS) regulation of the immune response: LPS converts germfree mice to sensitivity to oral tolerance induction. J Immunol. 1982; 129:959-65). Dysregulation of regulatory immune responses caused by missing environmental stimuli in developed "clean countries" may be the underlining factor for the recent epidemiological increase of autoimmune diseases in genetically predisposed individuals. That is why, boosting the regulatory immune mechanisms with disease-relevant environmental antigens represents a new, promising and inexpensive strategy for secondary but also primary prevention of type 1 diabetes.

Type 1 diabetes is a disease with very good animal models widely used in the research of the development of the disorder. Among several models, the NOD (Non Obese Diabetic) mouse is far the most used model. It resembles the human Type 1 diabetes closely including the insulitis preceeding the clinical disease. (Buschard K, Thon R: Diabetic animal models. In: Hau J, van Hoosier Jr GL (eds.): Hand-book of laboratory animal science. Second edition. CRC Press, 153-182, 2003). Several beta-cell autoantigens in type 1 diabetes and/or their immunodominant peptides are being tested as a vaccination against ongoing type 1 diabetes. There are also a number of earlier publications describing gluten-free diets preventing manifestation of type 1 diabetes in animal models. Surprisingly, the data of the invention herein shows that intranasal, mucosal vaccination with an environmental antigen - gliadin, and/or its components but not gluten, represent a novel and promising approach for prevention and/or early cure of type 1 diabetes in humans.

Schmid et al (Clinical Immunology; 2004, pages 108-118) discuss the influence of dietary gluten, vitamin D3, and fish-oil on the incidence of autoimmune diabetes. Schmid et al suggest that there is a link between dietary wheat and barley proteins and the development of autoimmune diabetes. Schmid et al only focus on the effect of dietary gluten on the natural history of diabetes in NOD mice. Due to the complexity of animal diets, it is very difficult to dissect this topic to get answers on the identity and mechanisms of dietary components. Various diets with modified content of grain-protein modify diabetes incidence in animal models, but this could be due to changes in gut microflora or due to other, even non-protein components within the chloroform-ethanol fraction of wheat flour/gluten. In fact, Schmid et al state that supplementing the diet with gliadin did not restore the diabetogenic potential of the standard diet.

The prior art does not suggest the use of gliadin as a therapeutical agent for prevention or early cure of diabetes.

### SUMMARY OF THE INVENTION

The invention herein discloses that intranasal, or other mucosal administration of gliadin prevents development of type 1 diabetes. Gliadin or various fractions of gliadin may be useful for prevention or early cure of diabetes. A suitable fraction may be a gliadin peptide, or deamidated, tranglutaminase (tTG)-treated gliadin or its fractions or fragments of gliadin generated by other enzyme digestions or e.g. a fraction disclosed in Maurano et al (Maurano F, Siciliano RA, De Giulio B, Luongo D, Mazzeo MF, Troncone R, Auricchio S, Rossi M. Intranasal administration of one alpha gliadin can downregulate the immune response to whole gliadin in mice. Scand J Immunol. 2001; 53:290-295).

Thus, the invention provides in one embodiment gliadin or a derivative thereof selected from the group consisting of alpha gliadin, beta-gliadin, gamma-gliadin, deamidated gliadin, and tissue tranglutaminase (tTG)-treated gliadin for use in preventing or treating diabetes by inducing tolerance by gliadin in a patient.

I n accordance with the present invention gliadin or a derivative thereof is preferably administered intranasally. However, other routes of administration, especially a mucosal administration, are also applicable, such as where gliadin or derivative thereof is administered as a suppository or capsule. Preferably, the suppository or capsule has an enteric coating for release of gliadin or derivative thereof in the bowel of the patient. Alternatively, the gliadin or derivative thereof is administered sublingually, subcutaneously or intravenously. Preferably the gliadin or a derivative thereof is administered as intranasal spray or sublingual tablets, however, application routes of the present invention could be any possible route of mucosal administration, also including sublingual, rectal, urethral, and vaginal application.

It should be emphasized that the present invention encompasses the prevention of diabetes in subject with no special risk (background population) as well as subjects with non-specific and specific (such as tissue type or family) risk for developing diabetes as well as subjects which are antibody positive or have reduced beta cell function.

It is to be understood that when reference is made to diabetes in accordance with the present invention this not only includes Type 1 and Type 2 diabetes but also early stages thereof as well as Latent Autoimmune Diabetes Adult (LADA) (Zimmet PZ, Tuomi T, Mackay IR, Rowley MJ, Knowles W, Cohen M, Lang DA. Latent autoimmune diabetes mellitus in adults (LADA): the role of antibodies to glutamic acid decarboxylase in diagnosis and prediction of insulin dependency. Diabet Med. 1994; 11:299-303), Maturity-Onset Diabetes of the Young (MODY) (Herman WH, Fajans SS, Ortiz FJ, Smith MJ, Sturis J, Bell GI, Polonsky KS, Halter JB. Abnormal insulin secretion, not insulin resistance, is the genetic or primary defect of MODY in the RW pedigree. Diabetes 1994; 43:40-46), impaired glucose tolerance (IGT) (Expert Committee on Classification of Diabetes Mellitus, Diabetes Care 22 (Supp. 1) S5 (1999)), impaired fasting glucose (IFG) (Charles MA, Fontbonne A, Thibult N, Warnet JM, Rosselin GE, Eschwege E. Risk factors for NIDDM in white population. Paris prospective study. Diabetes 1991; 40:796-799), gestational diabetes (Metzger BE. Summary and recommendations of the Third International Workshop-Conference on Gestational Diabetes Mellitus. Diabetes 1991; 40:197-201), and metabolic syndrome X.

The present invention also provides a therapeutic system for use in preventing or treating diabetes in a patient, the system comprising gliadin or a derivative thereof selected from the group consisting of alpha gliadin, beta-gliadin, gamma-gliadin, deamidated gliadin, and tissue tranglutaminase (tTG)-treated gliadin, said therapeutic system is an aerosol, liquid, dry powder, suppository or capsule.

This therapeutic system may be designed for mucosal administration such as intranasal, oral, sublingual, rectal, urethral, or vaginal administration, and thus formulated as an aerosol, liquid, dry powder, suppository or capsule. In a preferred embodiment the therapeutic system gliadin or derivative thereof as defined above is in a dry powder intranasal composition comprising hydroxypropylmethylcellulose.

Moreover, the present invention provides a device comprising the dry powder composition comprising gliadin and hydroxypropylmethyl-cellulose, the device being suitable for delivering the composition to the nasal tract.

It should be understood that in addition to the ingredients particularly mentioned above the formulations of this invention may include other agents conventional in the art having regard to the type of formulation in question, for example those suitable for oral administration may include flavouring agents.

### BRIEF DESCRIPTION OF THE DRAWINGS

**Figure 1** shows the effect of multiple intranasal administrations of gliadin compared to control protein-ovalbumin (OVA), on cumulative diabetes incidence in NOD/Bom female mice (n=16) by 190 days. 4-week-old NOD females received 5 intranasal immunizations (every other day).
**Figure 2** shows the effect of multiple intranasal administrations of gliadin, ovalbumin (OVA), gluten, IL-10 and gluten+IL-10 on cumulative diabetes incidence in NOD/Bom female mice (n=16) by 190 days. 4-week-old NOD females received 5 intranasal immunizations (every other day).
**Figure 3** shows the grade for insulitis scoring , as follows: 0, Normal islet; 1, intact islet with few scattered mononuclear cells in the suroundings; 2, peri-insulitis; 3, moderate insulitis (50% of the islet infiltrated); 4, severe insulitis (>50% of the islet infiltrated).

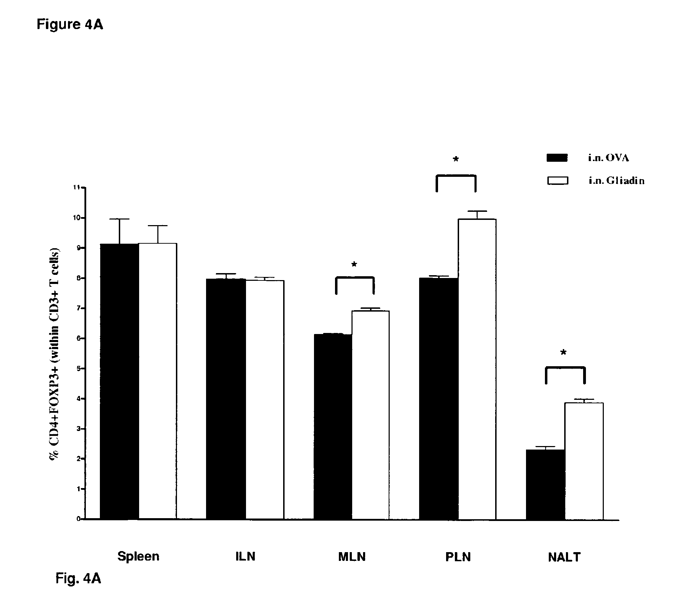

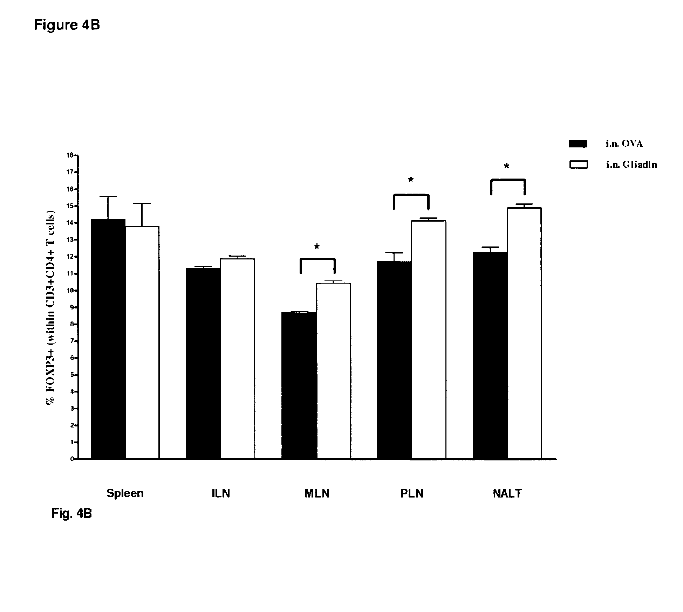

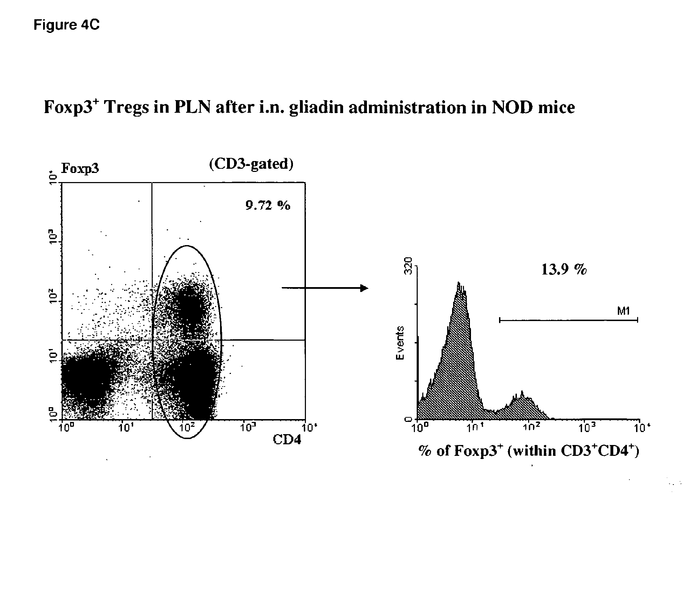

**Figure 4** shows A/ the proportion of regulatory CD4⁺FOXP3⁺ cells within T (CD3-gated) cells in mucosal (NALT - Nasal associated lymphoid tissue, MLN - Mesenteric lymph nodes, PLN - pancreatic lymph nodes) and non-mucosal compartments (Spleen, ILN - inguinal lymph nodes) after i.n. gliadin or OVA vaccination in 4 weeks old NOD female mice. B/ Proportion of regulatory CD4⁺Foxp3⁺ cells (Tregs) expressed as percentage of CD3⁺CD4⁺ helper T cell subset; lymphoid organs as ad A. C/ Panel C shows example of FACS analysis of CD4⁺Foxp3⁺ T cells in PLN of NOD mice i.n. treated with gliadin. Individual measurements were performed on cells pooled from 2-3 experimental animals. An example of two independent experiments.

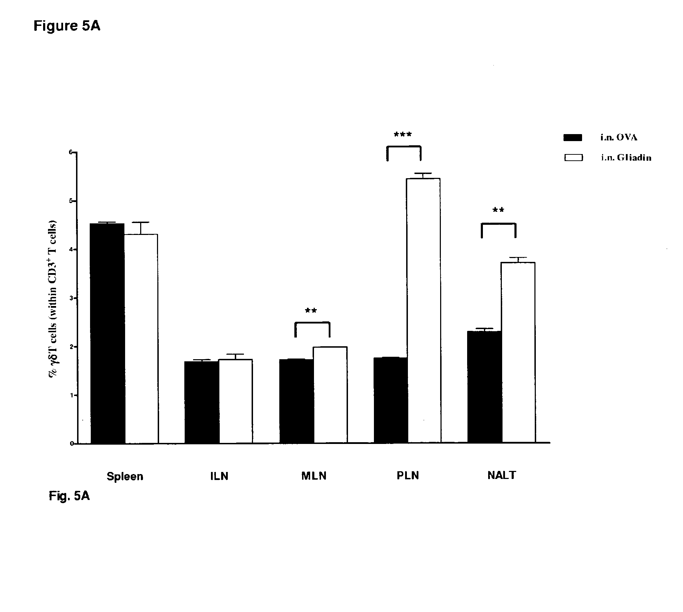

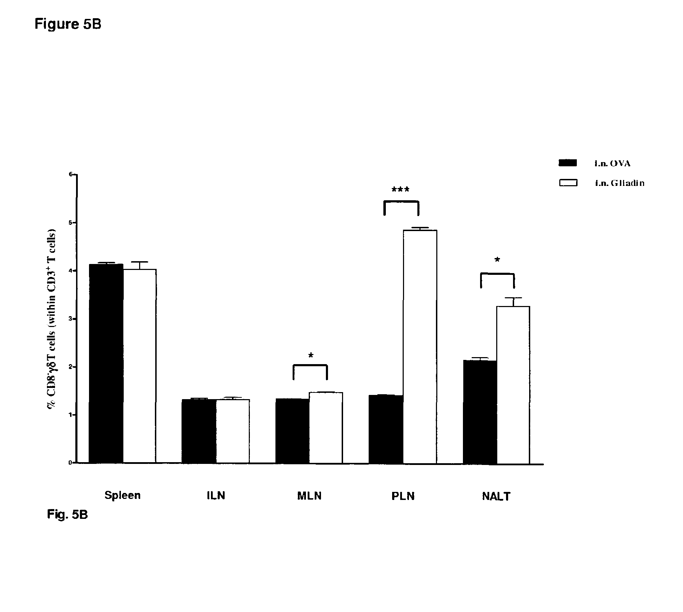

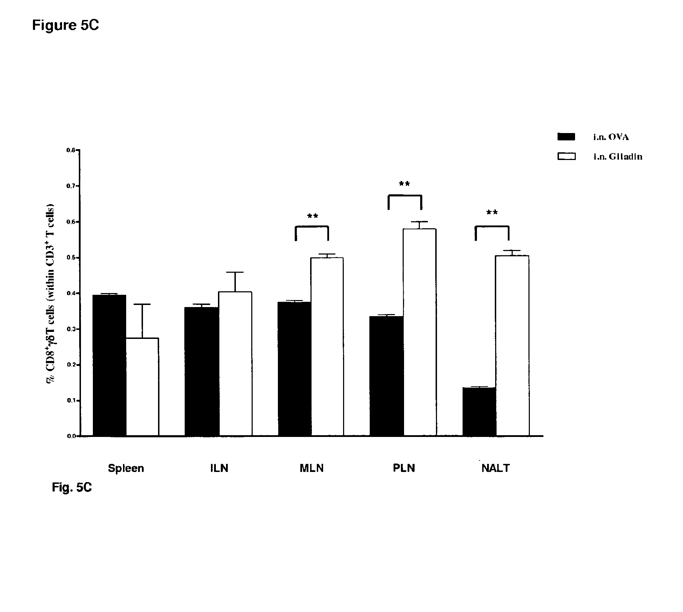

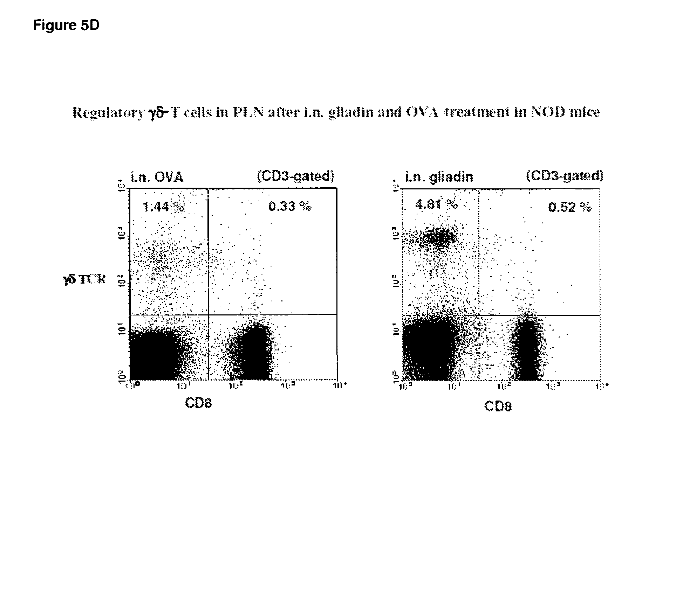

**Figure 5** shows the effect of i.n. administration of gliadin on frequency of gamma/delta T cells. A/ Proportion of regulatory gamma/delta T cells within T (CD3-gated) cells in mucosal (NALT - Nasal associated lymphoid tissue, MLN - Mesenteric lymph nodes, PLN - pancreatic lymph nodes) and non-mucosal compartments (Spleen, ILN - inguinal lymph nodes) after i.n. gliadin or OVA vaccination in 4 weeks old NOD female mice. B/ Panel B and C show further analysis of the i.n. gliadin induced regulatory gamma/delta T cells according to their CD8 expression. B/ Proportion of CD8⁺gamma/delta T cells expressed as percentage of CD3⁺ gamma/delta T cells. C/ Proportion of CD8⁻gamma/delta T cells expressed as percentage of CD3⁺ gamma/delta T cells. D/ Panel C shows example of FACS analysis of gamma/delta T cells (CD3-gated) in MLN of NOD mice i.n. treated with gliadin and OVA. Individual measurements were performed on cells pooled from 2-3 experimental animals. An example of two independent experiments.

### DETAILED DESCRIPTION OF THE INVENTION

Using 4-week-old NOD female mice the present inventors have surprisingly shown that five intranasal administrations at age of 4-5 weeks significantly (p=0.0038) reduced penetrance of diabetes from 81% to 44%. This is for the first time, an environmental antigen, which may have etiological role in the development of diabetes in genetically predisposed individuals, has been successfully applied in the disease prevention. Other objects and features of the inventions will be more fully apparent from the following disclosure and appended claims.

The formulation used in the present invention is preferably a nasal spray, or nasal drops, so that the administration of gliadin or fragments thereof can be effected on an intranasal route. The spray (or the drops) according to the invention is useful, in particular, for vaccination of diabetes type 1. The dose should be in the range of 1-100µl (0,5%) gliadin or fragments thereof, but preferably 10 µl (0,5%) one or several times, with possibly one or several booster doses at the similar level.

Formulations used in the present invention may further comprise a thickening agent such as a gum or starch; a disintegrant, such as sodium starch glycolate or cross-linked povidone; a release agent such as magnesium stearate; an emulsifying agent; a surfactant; pharmaceutically acceptable excipients; anti-caking agents; granulating agents; preservatives; such colorants as may be desired or any combination thereof.

Powder formulations used in the present invention do not include components which are often used in intranasal compositions (dry powders or solutions) which can cause irritation or affect ciliary movement, for example, solvents, such as propylene glycol, absorption enhancers, such as cyclodextrins or glycosides, or mucoadhesives such as chitosan. The use of such additives can be undesirable, as they can cause discomfort and interfere with the normal functioning of the nose, which can adversely affect breathing.

Formulations used in the present invention may further comprise a flavouring or signaling agent or additive such as menthol, mint, spearmint, peppermint, eucalyptus, lavender, citrus, lemon, lime, or any combination thereof. The inclusion of such flavouring or signaling agents in the composition can provide the patient with pleasant sensory feedback upon use, which allows the patient to recognize that administration has occurred, and may aid the patient's recollection of administration. Such factors can improve patient compliance and provide a positive psychological effect.

In certain embodiments of the present invention, the combination of the HPMC and therapeutic agent (gliadin or derivative thereof) is provided for sequential or simultaneous administration. The HPMC and therapeutic agent may be included together in a single preparation. Alternatively, the HPMC and therapeutic agent may be provided in separate preparations, for sequential administration. Where administration is sequential, the HPMC may be administered before or after the therapeutic agent, or both. Similarly, the therapeutic agent may be administered before or after the HPMC.

Where the powdered HPMC is included in the same preparation as the therapeutic agent, this preparation is preferably in the form of a powder. The therapeutic agent may, however, be in any form and is preferably in a form suitable for nasal administration, such as in the form of a powder, liquid or cream or gel.

A device which is suitable for dispensing the formulations used in the present invention is disclosed in British Patent Publication No. 2378176A. The bottles disclosed therein use a very simple mechanism for restricting the amount of powder which is dispensed. Whilst the amount of powdered cellulose delivered to the nasal tract in order to enhance natural mucus does not have to be precisely controlled, the administration of too much powder could potentially cause an uncomfortable blockage of the nasal tract and may even result in difficulty in breathing through the nose.

The formulations used in the present invention are preferably administered in amounts of between about 0.001 mg and about 10000 mg per nostril. Preferably, the dose is between about 0.05 mg to about 1000 mg, between 0.5 mg and about 500 mg, between about 1 mg and about 250 mg, between about 1 mg and about 100 mg, or between about 1 mg and about 50 mg.

The formulations used in the invention could also be sublingual. The dose should be in the range of 1-100µl (0.5%-1 %) gliadin or fragments thereof, but preferably 10 µl (0.5%) one or several times, with possibly one or several booster doses at a similar level.

Examples of fragments of gliadin in this invention include also: alpha gliadins, beta-gliadins, gamma-gliadins, deamidated gliadin or deamidated gliadin fragments thereof , also tissue transglutaminase (tTG)-treated gliadin and tissue transglutaminase(tTG)-treated gliadin fragments thereof as well as fragments of gliadin generated by enzyme digestion such as with pepsin, pepsin-trypsin, DPP IV, and other enzymes as well as, chemically modified gliadin, acetic acid and/or ethanol extract of gliadin, gliadin fragments, as well as gliadin peptides in both natural and deamidated forms.

Adjuvants are normally used to enhance the absorption of the antigen as well as to enhance the immune-stimulating properties of the antigen.

Examples of suitable adjuvants are chemochines, cytokines (IL-10, TGF-beta, INF-gamma), zonula occludens toxin, heat shock proteins, cholera toxin B subunit, liposomes, Lipovax C, CpG, microbial lysates, plant glucans, ISCOMATRIX etc.

In addition to intranasal spray and sublingual tablets, application routes of the invention herein could be any possible route of mucosal administration.

Further there are several possible variations of the invention in the prevention and treatment of disease in many of the sub-groups of diabetes and pre-diabetes, including LADA, Moody and gestational diabetes.

### EXAMPLE 1

### Preparation of gliadin fragments (pepsin-digest fragment/peptides)

Pepsin fragments of gliadin (Fluka, Sigma) were prepared using pepsin agarose gel (ICN Biomedicals, OH, USA). Gliadin was dissolved in acidified (0,2 % acetic acid, Merck, NJ, USA) saline solutions. 2,5 ml of 1% gliadin were incubated with 2,5 ml of pepsin-agarose gel (pH 3,2) on a shaker at 37° C for 1 h. The amount of gliadin for enzyme digestion was increased by 20 % to compensate for loses due to the procedure, as determined in previous set-up experiments. Pepsin-agarose was removed by centrifugation (2000 x g, 10 min, 4 °C), which has lead to rapid termination of the enzymatic cleavage. Supernatants were then re-centrifuged at 12000 x g, 10 min, 4 °C, pH of soluble gliadin fragments adjusted to neutral, and aliquots stored frozen at -20°C.

### EXAMPLE 2

### Animals

NOD/Bom mice controlled according to FELASA recommentations for health monitoring (W. Nicklas, P. Baneux, R. Boot, T. Decelle, A. Deeny, M. Fumanelli, and B. Illgen-Wilcke. Recommendations for the health monitoring of rodent and rabbit colonies in breeding and experimental units. Laboratory Animals 36 (1):20-42, 2002) were obtained from Taconic Europe A/S, Ry, Denmark. Mice had free access to acidified drinking water and were fed standard Altromin 1324 diet (Altromin, Lage, Germany). All animals were maintained in a barrier protected facility at the Faculty of Life Science, University of Copenhagen, and experiments were carried out according to the principles of the national laws on Animal Experimentation, which adhere to the EU directive 86/609 and are equivalent to US animal care (NIH publication no. 85-23, revised 1985).

### EXAMPLE 3

### Immunization scheme 1

Four weeks old NOD female mice were immunized 5 times during period of 9 days (every other day).

### Immunization scheme 2

Three sets of treatments starting at 4 weeks of age. Each treatment set consists of 5 single intranasal immunizations every other day. There are 10 days of break between these sets of treatments.

### EXAMPLE 4

### Intranasal immunization of gliadin and monitoring of diabetes

Non-anesthetized 3-4 weeks old NOD female mice were given 50 µg of ovalbumin (Sigma, St. Louis, MO), gliadin, and/or gluten (Sigma, St. Louis, MO), in a total volume of 10 µl (5 µl per nostril). Gliadin, gluten as well as ovalbumin were dissolved in acidified (0.2% acetic acid) saline solution. IL-10 (R&D Systems, Minneapolis, MN USA) was administered at 0.02 µg/10 µl of PBS. Animals were immunized 5 times every other day. Experimental groups receiving an antigen (gluten) in combination with IL-10, were first given 0.02 µg of IL-10 in 10 µl of PBS, 3 hours later 50 µg of gluten dissolved in acidified (0.2% acetic acid) saline.

### Result

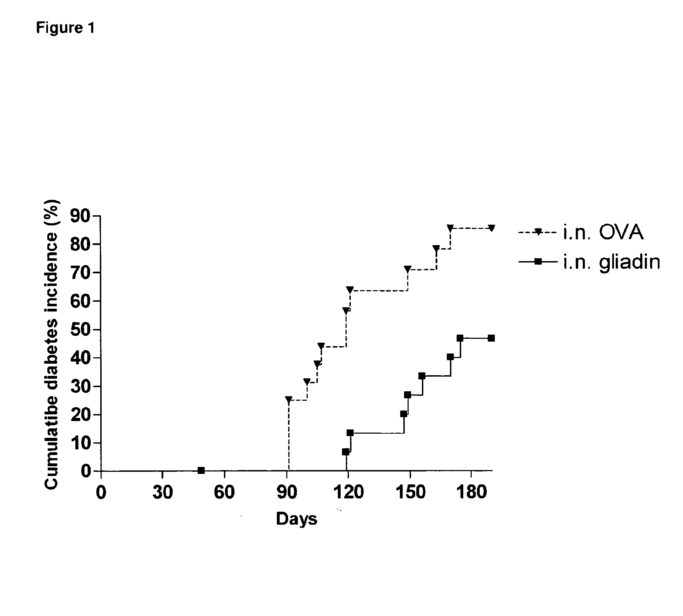

As shown in Fig.1, five intranasal administrations of gliadin (50 µg) delayed and significantly decreased diabetes incidence in comparison to NOD mice treated with a control protein, ovalbumin (OVA) (p=0.0038 by log rank test). Compared to control ovalbumin-treated mice, intranasal gliadin treatment delayed first diabetes onset by 30 days and reduced diabetes incidence from 81 % to 44 % by 190 days of age.

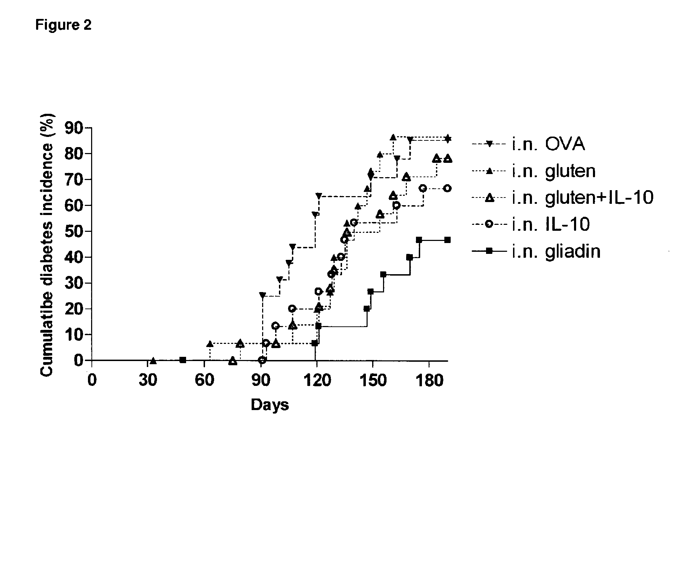

As shown in Fig.2 NOD/Bom mice treated intranasally with gluten, IL-10, and/or gluten+IL-10 did not display statistically significant reduction in diabetes incidence compared to OVA-treated mice (Fig.2). Interestingly, crude wheat gluten had no effect on diabetes prevention in NOD mice, and similarly to OVA-treated mice, also led to diabetes penetrance at 81% by 190 days. Thus, the difference between gliadin- and gluten-treated NOD female mice was also significant at p=0.0057 (log rank test).

Similarly to OVA-treated mice, 81% gluten-treated, 63% IL-10 treated and 69% gluten+IL-10 treated NOD mice developed diabetes at 190 days of age. While there was no difference between OVA- and gluten-treated mice, IL-10-treated mice showed slightly lower diabetes frequency (81% vs. 63%, ns). Thus, the slightly decreased diabetes incidence in gluten+IL-10-treated NOD female mice (69% at 190 days) might be on account of IL-10 tolerogenic effect.

### EXAMPLE 5

### Reduction of insulitis by intranasal administration of gliadin. Histology and insulitis scoring

Five animals per group at age of 90 days were used for insulitis scoring and immunohistochemistry. None of these animals became diabetic, i. e. displayed blood glucose levels > 12 mmol/l. For diabetes incidence studies, 16 mice per group were monitored for 190 days. NOD mice were inspected daily for diabetes and from 70 days of age screened weekly for glycemia with Glucometer FreeStyle mini (Hermedico). Diagnosis of diabetes was based on two consecutive positive blood glucose readings >12mmol/l during three days. The date of the first positive glycemia reading was used as the onset of diabetes.

Mice were killed by CO2 asphyxia, pancreata removed and cut longitudinally. One half of the pancreas was fixed in 4 % formaldehyde, embedded in paraffin and stained with H&E for insulitis scoring, whereas the other half was used for immunohitochemistry. The grade for insulitis scoring was as follows: 0, Normal islet; 1, intact islet with few scattered mononuclear cells in the surroundings; 2, peri-insulitis; 3, moderate insulitis (up to 50% of the islet infiltrated); 4, severe insulitis (>50% of the islet infiltrated).

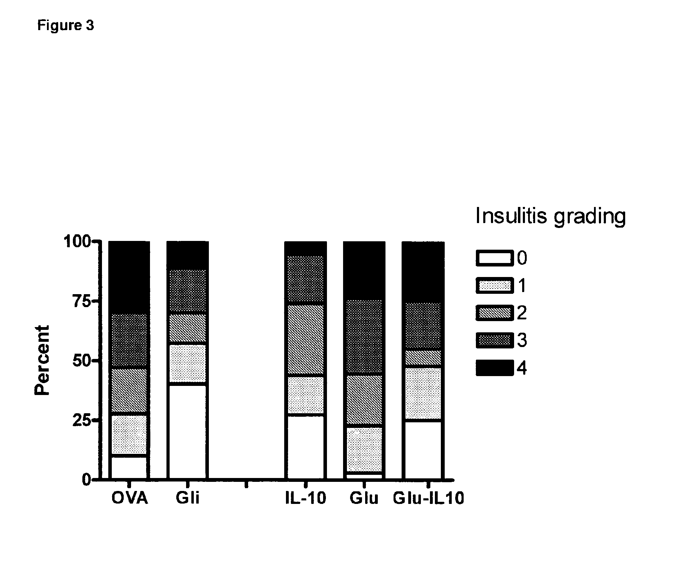

### Result (Figure 3)

Insulitis scoring was performed on H&E stained pancreata from non-diabetic NOD females (n=5) at age of 90 days. For gliadin, OVA, gluten, IL-10 and gluten+IL-10 treated groups of NOD mice, insilitis was found to be present at levels that can be seen from Figure 3. The insulitis score was 1.44±0.13, 2.44±0.13; 2.54±0.11; 1.60±014 and 1.97±0.13 for gliadin, OVA, gluten, IL-10 and gluten+IL-10-treated groups, respectively. Gliadin-treated NOD females (insultis score 1.44±0.13) revealed statistically significant, less destructive insulitis compared to OVA (2.44±0.13, p<0.001), gluten (2.54±0.11; p<0.001) and to lesser extent also to gluten+IL10 (1.97±0.13, p<0.05) treated groups. Similarly IL-10 treated groups showed significantly lower insulits score compared to OVA (2.44±0.13, p<0.001) and gluten (2.54±0.11; p<0.001) treated groups. Gluten+IL10-treated group also displayed less severe insulits compared to gluten-treated group (p<0.05). In accord with the diabetes incidence data, insulitis scoring revealed a beneficial effect of gliadin but not gluten on islet preservation. Similarly IL-10 (IL-10 and gluten+IL10 groups) showed a beneficial effect on islet preservation. The less severe insulitis in the gluten+IL-10 group is thus most likely due to the effect of IL-10. In conclusion, the insulitis data very closely paralleled and confirmed the diabetes incidence data (Fig 1 and 2) in an independent experiment (animals, immunizations). Thus, this result shows that gliadin is preferred to gluten as an antigen for immunization against type 1 diabetes.

### EXAMPLE 6

### Statistics

The cumulative diabetes incidence was assessed using the Kaplan-Meier estimation and log-rank test was used for comparisons between groups. Other results are expressed as mean ±SEM, and the level of significance (p<0.05) was assayed by two-sample analysis (unpaired *t*-test).

### EXAMPLE 7

### Induction of regulatory T cells by intranasal administration of gliadin. FACS analysis.

Four weeks old NOD female mice (4-5 animals per group) were immunized i.n. with OVA or gliadin according to the protocol as described in Example 4. Cell suspension were prepared from NALT (Nasal Associated Lymphoid Tissue), pancreatic (PLN), mesenteric (MLN) and inguinal (ILN) lymph nodes, and spleen in RPMI medium supplemented with 10% FCS. For intracellular detection of cytokines after "in vitro" stimulation cells were cultured and stimulated with PMA/ionomycin for 3h in complete RPMI medium in presence of Golgi-stop. Cultures of unstimulated cells were used as controls. In other experiments isolated cells were kept on ice and used for surface (30 min) and intracellular (anti-Foxp3) staining (30 min) with directly conjugated mAbs (BD, E-Bioscience). All surface staining was done on live cells in PBS containing BSA and NaN3 (Sigma). For intracellular detection (e.g. cytokines, Foxp3 staining), live cells were first stained for surface markers, then permeabilized with cytofix/cytoperm kit and stained for anti-Foxp3 PE-conjugated mAb (E-Bioscience) for 30 min and fixed with a 4% paraformaldehyde solution. Fluorescence was measured and cells analyzed on FACScan (BD Bioscience).

### Results

As shown in Fig. 4, i.n. administration of gliadin to NOD mice at 4 weeks of age lead to induction of CD4⁺Foxp3⁺ T (CD3⁺) regulatory T cells (Tregs). These cells were specifically induced by i.n. administration of gliadin compared to the control protein (ovalbumin, OVA) at the site of the antigen administration - in the NALT (Nasal Associated Lymphoid Tissue) and in the mucosal draining lymph nodes of the pancreas - pancreatic (PLN) and mesenteric (MLN) lymph nodes. Thus, statistically significant increase of CD3⁺CD4⁺Foxp3 Tregs was found in NALT (p=0.013), MLN (p=0.014) and PLN (p=0.019) after i.n. gliadin vaccination (Fig. 4A). "In vitro" restimulation with PMA/ionomycin revealed an increased proportion of IL-10 producing cells in PLNs and MLNs of gliadin-treated mice. These data are significant also when expressed as proportion of CD4⁺ helper T cells (NALT, p=0.019; MLN, p=0.011; PLN, p=0.049), Fig. 4B. I.n. gliadin-induced increase of Foxp3⁺ Tregs was not found in other non-mucosal lymphoid organs such as spleen and control systemic, inguinal lymph nodes (ILN), Fig. 4. Collectively, induction of Foxp3+ Tregs at the site of immunization and in draining lymph nodes of the pancreas was significant both when expressed as proportion of total T cells (CD3-gating, Fig. 4A) or proportion of CD3⁺CD4⁺T helper cells (Fig 4B).

I.n. administration of gliadin led to a mucosal-specific induction of gamma/delta T (CD3⁺) cells in NOD mice. Thus, i.n. gliadin administration, that substantially prevented development of diabetes in 4-week-old NOD female mice (Example 4), led to a significantly increased frequency of regulatory gamma/delta T cells in NALT (p=0.004), MLN (p=0.004) and PLN (p=0.001), but not in non-mucosal lymphoid organs such as spleen and control systemic, inguinal lymph nodes (ILN), Fig. 5. The increased frequency of gamma/delta T (CD3-gated) cells was found both within gamma/delta T expressing CD8 marker (NALT, p=0.002; MLN, p=0.008; and PLN, p=0.007) as well as within and CD8⁻ gamma/delta T cell subset (NALT, p=0.027; MLN, p=0.013; and PLN, p=0.001), Fig. 5B and C. In MLN and NALT, there was a shift towards CD8⁺ expression within the gamma/delta T (CD3⁺) cells in glaidin vs. OVA treated animals.

In conclusion, i.n. administration of gliadin, that prevents development of diabetes in NOD mice, is characterized by induction of regulatory CD4⁺Foxp3⁺ and gamma/delta T cells at the mucosal immunization site (NALT) and at mucosal lymph nodes (PLN, MLN) that drain the disease target organ in type 1 diabetes - the pancreas. These cells and their cytokine profiles may represent cellular basis for diabetes protection by i.n. gliadin vaccination.

### EXAMPLE 8

### Nasal preparation

A nasal preparation comprised of gliadin or fragments thereof can take a variety of forms for administration for example spray, drops, gel, ointment, cream, powder or suspension, using a dispenser or other device as needed. A variety of dispensers and delivery vehicles are known in the art, including single-dose ampoules, atomizers, nebulizers, pumps, nasal pads, nasal sponges, nasal capsules, and the like.

More generally, the preparation can take a solid, semi-solid, or liquid form. In the case of a solid form, the components may be mixed together by blending, tumble mixing, freeze-drying, solvent evaporation, co-grinding, spray-drying, and other techniques known in the art.

A semi-solid preparation suitable for intranasal administration can take the form of an aqueous or oil-based gel or ointment. For example, gliadin or fragments thereof can be mixed with microspheres of starch, gelatin, collagen, dextran, polylactide, polyglycolide, or other similar materials that are capable of forming hydrophilic gels. The microspheres can be loaded with drug, and upon administration form a gel that adheres to the nasal mucosa.

Preferably, the nasal preparation is in liquid form, which can include an aqueous solution, an aqueous suspension, an oil solution, an oil suspension, or an emulsion, depending on the physicochemical properties of the composition components. The liquid preparation is administered as a nasal spray or as nasal drops, using devices known in the art, including nebulizers capable of delivering selected volumes of formulations as liquid-droplet aerosols. For example, a commercially available spray pump with a delivery volume of 50 µL or 100 µL is available from, for example, Valois (Congers, N.Y.) with spray tips in adult size and pediatric size.

The liquid preparation can be produced by known procedures. For example, an aqueous preparation for nasal administration can be produced by dissolving, suspending, or emulsifying gliadin or fragments thereof in water, buffer, or other aqueous medium, or in a oleaginous base, such as a pharmaceutically-acceptable oil like olive oil, lanoline, silicone oil, glycerine fatty acids, and the like.

It will be appreciated that excipients necessary for formulation, stability, and/or bioavailability can be included in the preparation. Exemplary excipients include sugars (glucose, sorbitol, mannitol, sucrose), uptake enhancers (chitosan), thickening agents and stability enhancers (celluloses, polyvinyl pyrrolidone, starch, etc.), buffers, preservatives, and/or acids and bases to adjust the pH, and the like. The pH should be <7 or >7, because neutral pH decreases the stability of gliadin or fragments thereof.

### EXAMPLE 9

### Sublingual preparation

Non-compressed tablets may be used in the present invention. A solid vaccine formulation is a fast-dissolving, non-compressed tablet suitable for buccal or sublingual administration. Examples of fast dissolving, non-compressed tablets are those disclosed in US-A-5,648,093, WO 00/51568, WO 02/1 3858, WO 99/21579, US-A-4,371,516, EP-278 877, WO 2004/047794. Preferred fast dissolving, non-compressed tablets are those produced by freeze-drying. Preferred matrix forming agents are fish gelatine and modified starch.

The non-compressed tablet may in addition to gliadin or fragments thereof include any conventional tablet-forming agent or excipient, such as adjuvants, antacids, diluents, enhancers, mucoadhesive agents, flavouring agents, taste masking agents, preservatives, antioxidants, surfactants, viscosity enhancers, colouring agents, pH modifiers, sweeteners etc. These excipients are all selected in accordance with conventional pharmaceutical practice in a manner understood by the persons skilled in the art of formulating allergen vaccines.

Preferably, the tablet contains a protein stabilizing agent. Examples of protein stabilising agents are polyethylene glycols (PEG), e.g. PEG300, PEG400, PEG600, PEGI000, PEGI500, PEG3000, PEG3050, PEG4000, PEH6000, PEG20000 and PEG35000; amino acids, such as glycine, alanine, arginine; mono-, di and tri-saccharides, such as trehalose and sucrose; polyvinylalcohol (PVA); polyoxyethylene sorbitan fatty acid esters (polysorbates, tweens or span); human serum albumin (HSA); bovine serum albumin (BSA). Preferably, PEG is used as protein stabilising agent. In addition to being a protein stabiliser, PEG is believed to confer the property of elasticity to the matrix of the dosage form.

Suitable colouring agents include red, black and yellow iron oxides and FD & C dyes such as FD & C blue No. 2 and FD & C red No. 40. Suitable flavouring agents include mint, raspberry, liquorice, orange, lemon, grapefruit, caramel, vanilla, cherry and grape flavours and combination of these. Suitable pH modifiers include citric acid, tartaric acid, phosphoric acid, hydrochloric acid and maleic acid. Suitable sweeteners include aspartame, acesulfame K and thaumatic. Suitable taste-masking agents include sodium bicarbonate, ion-exchange resins, cyclodextrin inclusion compounds, adsorbates or microencapsulated actives.

## Claims

1. Gliadin or a derivative thereof selected from the group consisting of alpha gliadin, beta-gliadin, gamma-gliadin, deamidated gliadin, and tissue tranglutaminase (tTG)-treated gliadin for use in preventing or treating diabetes by inducing tolerance by gliadin in a patient.

2. Gliadin or a derivative thereof as defined above for use according to claim 1, wherein gliadin or a derivative thereof, is formulated to be administered mucosally.

3. Gliadin or a derivative thereof as defined above for use according to claim 1, wherein gliadin or a derivative thereof is administered as a suppository or capsule.

4. Gliadin or a derivative thereof as defined above for use according to claim 3, wherein the suppository or capsule has an enteric coating for release of gliadin or derivative thereof in the bowel of the patient.

5. Gliadin or a derivative thereof as defined above for use according to claim 1 wherein the gliadin or a derivative thereof is administered subcutaneously or intravenously.

6. Gliadin or a derivative thereof selected from the group consisting of alpha gliadin, beta-gliadin, gamma-gliadin, deamidated gliadin, and tissue tranglutaminase (tTG)-treated gliadin, for use in preventing or treating diabetes in a patient, wherein the patient is administered intranasally with gliadin or a derivative thereof.

7. A therapeutic system for use in preventing or treating diabetes in a patient, the system comprising gliadin or a derivative thereof selected from the group consisting of alpha gliadin, beta-gliadin, gamma-gliadin, deamidated gliadin, and tissue tranglutaminase (tTG)-treated gliadin, said therapeutic system is an aerosol, liquid, dry powder, suppository or capsule.

8. The therapeutic system for use according to claim 7, wherein gliadin or derivative thereof as defined above is in a preparation for systemic administration.

9. The therapeutic system for use according to claim 7, wherein gliadin or derivative thereof as defined above is in a preparation for oral administration.

10. The therapeutic system for use according to claim 7, wherein gliadin or derivative thereof as defined above is formulated as a suppository or capsule.

11. The therapeutic system for use according to claim 7, wherein gliadin or derivative thereof as defined above is in a preparation for intranasal administration in a liquid or aerosol form as an aerosol-spray or nasal drops.

12. The therapeutic system for use according to claim 7, wherein gliadin or derivative thereof as defined above is in a dry powder intranasal composition comprising hydroxypropylmethylcellulose.

13. A device comprising the dry powder composition of claim 12, the device being suitable for delivering the composition to the nasal tract.

## Patentansprüche

1. Gliadin oder ein Derivat davon ausgewählt aus der Gruppe, bestehend aus alpha-Gliadin, beta-Gliadin, gamma-Gliadin, deamidiertes Gliadin und mit Gewebe-Transglutaminase (tTG)-behandeltes Gliadin zur Verwendung in der Vorbeugung oder Behandlung von Diabetes durch Induzieren einer Toleranz in einem Patienten durch Gliadin.

2. Gliadin oder ein Derivat davon gemäß vorstehender Definition zur Verwendung nach Anspruch 1, wobei Gliadin oder ein Derivat davon zur Verabreichung über die Schleimhaut formuliert sind.

3. Gliadin oder ein Derivat davon gemäß vorstehender Definition zur Verwendung nach Anspruch 1, wobei Gliadin oder ein Derivat davon als Zäpfchen oder Kapsel verabreicht werden.

4. Gliadin oder ein Derivat davon gemäß vorstehender Definition zur Verwendung nach Anspruch 3, wobei das Zäpfchen oder die Kapsel eine magensaftresistente Beschichtung zum Abgeben von Gliadin oder einem Derivat davon im Darm des Patienten aufweist.

5. Gliadin oder ein Derivat davon gemäß vorstehender Definition zur Verwendung nach Anspruch 1, wobei Gliadin oder ein Derivat davon subkutan oder intravenös verabreicht werden.

6. Gliadin oder ein Derivat davon ausgewählt aus der Gruppe, bestehend aus alpha-Gliadin, beta-Gliadin, gamma-Gliadin, deamidiertes Gliadin und mit Gewebe-Transglutaminase (tTG)-behandeltes Gliadin zur Verwendung in der Vorbeugung oder Behandlung von Diabetes bei einem Patienten, wobei dem Patienten Gliadin oder ein Derivat davon intranasal verabreicht werden.

7. Therapeutisches System zur Verwendung in der Vorbeugung oder Behandlung von Diabetes bei einem Patienten, wobei das System Gliadin oder ein Derivat davon ausgewählt aus der Gruppe, bestehend aus alpha-Gliadin, beta-Gliadin, gamma-Gliadin, deamidiertes Gliadin und mit Gewebe-Transglutaminase (tTG) behandeltes Gliadin umfasst und das therapeutische System ein Aerosol, eine Flüssigkeit, ein Trockenpulver, ein Zäpfchen oder eine Kapsel ist.

8. Therapeutisches System zur Verwendung nach Anspruch 7, wobei Gliadin oder ein Derivat davon gemäß vorstehender Definition sich in einem Präparat zur systemischen Verabreichung befinden.

9. Therapeutisches System zur Verwendung nach Anspruch 7, wobei Gliadin oder ein Derivat davon gemäß vorstehender Definition sich in einem Präparat zur oralen Verabreichung befinden.

10. Therapeutisches System zur Verwendung nach Anspruch 7, wobei Gliadin oder ein Derivat davon gemäß vorstehender Definition als Zäpfchen oder Kapsel formuliert sind.

11. Therapeutisches System zur Verwendung nach Anspruch 7, wobei Gliadin oder ein Derivat davon gemäß vorstehender Definition sich in einem Präparat zur intranasalen Verabreichung in Form einer Flüssigkeit oder eines Aerosols, wie in einem Aerosolspray oder Nasentropfen, befinden.

12. Therapeutisches System zur Verwendung nach Anspruch 7, wobei Gliadin oder ein Derivat davon gemäß vorstehender Definition sich in einer intranasalen Trockenpulverzusammensetzung, die Hydroxypropylmethylcellulose umfasst, befinden.

13. Vorrichtung, umfassend die Trockenpulverzusammensetzung nach Anspruch 12, wobei die Vorrichtung zur Abgabe der Zusammensetzung an den Nasentrakt geeignet ist.

## Revendications

1. Gliadine ou un de ses dérivés, sélectionné du groupe consistant de alpha gliadine, beta gliadine, gamma gliadine, gliadine deaminée et gliadine traité par la tissue transglutaminase (tTG) pour l'usage dans la prevention ou le traitement du diabète en induisant une tolérance par la gliadine chez un patient.

2. Gliadine ou un de ses dérivés comme définie auparavant pour l'usage selon la revendication 1, dans lequel la gliadine ou un de ses dérivés estformulé pour une administration muqueuse.

3. Gliadine ou un de ses dérivés comme définie auparavant pour l'usage selon la revendication 1, dans lequel la gliadine ou un de ses dérivés est administré sous forme de suppositoire ou de capsule.

4. Gliadine ou un de ses dérivés comme définie auparavant pour l'usage selon la revendication 3, dans lequel le suppositoire ou la capsule présentant un revêtement enterique pour la libération de la gliadine ou un de ses dérivés dans l'intestin du patient.

5. Gliadine ou un de ses dérivés comme définie auparavant pour l'usage selon la revendication 1, dans lequel la gliadine ou un de ses derives est administré par voie subcutanée ou intraveineuse.

6. Gliadine ou un de ses derives, selectionné du groupe consistant de alpha gliadine, beta gliadine, gamma gliadine, gliadine deaminée et gliadine traité par la tissue transglutaminase (tTG) pour l'usage dans la prevention ou le traitement du diabète chez un patient, dans lequel le patient est administere par voie intranasale avec de la gliadine ou un de ses derives.

7. Un système thérapeutique pour prévenir ou traiter le diabète chez un patient, le système comprenant de la gliadine ou un de ses dérivés, selectionné du groupe consistant de alpha gliadine, beta gliadine, gamma gliadine, gliadine deaminée et gliadine traité par la tissue transglutaminase (tTG), le système étant un aérosol, un liquide, une poudre sèche, un suppositoire ou une capsule.

8. Le système thérapeutique pour l'usage selon la revendication 7, dans lequel la gliadine ou un de ses dérivés est dans une préparation pour l'administration systémique.

9. Le système thérapeutique pour l'usage selon la revendication 7, dans lequel la gliadine ou un de ses dérivés est dans une préparation pour l'administration orale.

10. Le système thérapeutique pour l'usage selon la revendication 7, dans lequel la gliadine ou un de ses dérivés est formulée sous forme de suppositoire ou de capsule.

11. Le système thérapeutique pour l'usage selon la revendication 7, dans lequel la gliadine ou un de ses dérivés est dans une préparation pour l'administration intranasale sous forme liquide ou en aérosol comme un aérosol-spray ou gouttes nasales.

12. Le système thérapeutique pour l'usage selon la revendication 7, dans lequel la gliadine ou un de ses dérivés est dans une composition intranasale de poudre sèche comprenant de la hydroxypropylméthylcellulose.

13. Un dispositif comprenant la composition de poudre sèche de la revendication 12, le dispositif étant apte à délivrer la composition aux voies nasales.
